# EUROPÄISCHE PATENTSCHRIFT

(11) **EP 1 140 276 B1**
(45) Veröffentlichungstag und Bekanntmachung des Hinweises auf die Patenterteilung: **21.02.2007**
(21) Anmeldenummer: 00975982.0
(22) Anmeldetag: 02.11.2000
(51) Int. Cl.: A61M 39/02

(54) **SINGLE-NEEDLE-PORT**
SINGLE-NEEDLE PORT
VOIE D'ACCES A AIGUILLE UNIQUE

(30) Priorität: 14.12.1999 DE 19960131
(43) Veröffentlichungstag der Anmeldung: 10.10.2001
(73) Patentinhaber: ESKA-medical Gesellschaft für Entwicklung und Vertrieb von medizinischen Implantat-Artikeln mbH, 23556 Lübeck (DE)
(72) Erfinder: GRUNDEI, Hans, 23558 Lübeck (DE); KRUG, Florian, 22529 Hamburg (DE); MÜNDER, Ulrich, 23558 Lübeck (DE)
(74) Vertreter: Fuchs Mehler Weiss & Fritzsche
(86) Internationale Anmeldenummer: PCT/EP2000/010780
(87) Internationale Veröffentlichungsnummer: WO 2001/043812

(56) Entgegenhaltungen:
- GB-A- 1 482 857
- US-A- 3 731 670
- US-A- 5 356 381
- US-A- 5 792 123

## Beschreibung

Die vorliegende Erfindung betrifft einen Single-Needle-Port, der subkutan implantierbar ist und zur Durchführung einer Hämodialyse dient. Derartige Single-Needle-Ports werden Patienten implantiert, die sich krankheitsbedingt mehrmals in der Woche einer Blutwäsche unterziehen müssen, beispielsweise wegen einer Niereninsuffizienz. Dabei wird dem Patienten über einen zentralen Gefäßzugang oder über einen Shunt eine Punktionskanüle gesetzt. Das häufige Setzen der Kanüle führt zu einer starken Belastung des Hautgewebes in weiten Bereichen. Daher ist man dazu übergegangen, derartigen Patienten einen sogenannten Port der vorliegenden Art zu implantieren, so daß lediglich eine Körperstelle bei der Vorbereitung der Hämodialyse wiederholt punktiert werden muß.

Rückseitig ist ein derartiger Single-Needle-Port dem Blutdruck des Patienten ausgesetzt. Dies bedeutet, daß dafür Sorge getragen werden muß, daß der Port, nachdem er durch die Punktionskanüle geöffnet worden ist und das Hämodialyseverfahren durchgeführt wurde, wieder sicher verschlossen wird, damit das unter dem eigenen Blutdruck stehende Blut des Patienten nicht aus dem Port austreten kann. Hier gibt es verschiedene Lösungsvorschläge, wie beispielsweise eine Membranlösung, bei der eine Punktionskanüle durch eine sich selbst wieder verschließende Membran in das Portinnere tritt.

Die Merkmale des Oberbegriffs des Anspruchs 1 sind aus US-A-5 356 381 bekannt.

Die bekannten Systeme haben sich insofern als nachteilig erwiesen, als die Anzahl der Öffnungen bzw. Schließungen des Ports begrenzt sind, was dann in einer erforderlichen Neuoperation und Implantation eines neuen Ports resultiert.

Vor diesem Hintergrund ist es die Aufgabe der vorliegenden Erfindung, hier Abhilfe zu schaffen, d. h. also einen Single-Needle-Port anzugeben, der seine Funktion praktisch ohne Verschleiß ausübt, d. h. also sich sehr viel öfter als bekannte Ports öffnen und schließen läßt.

Gelöst wird diese Aufgabe durch ein Single-Needle Port mit den Merkmalen des Anspruchs 1. Vorteilhafte Weiterbildungen ergeben sich aus den Unteransprüchen.

Demgemäß wird vorgeschlagen, daß das Verschlußelement des Ports gebildet ist aus einem die Portöffnung einfassenden Ring und aus einer Verschlußkugel, die einen größeren Durchmesser als die Portöffnung aufweist und die sich zu deren Abdichtung vom Portinneren her gegen eine dichte Fläche des Ringes anlegen kann und aus dieser Lage zur Freigabe des Lumens des Ports von einer in die Portöffnung zu führenden Punktionskanüle verschoben werden kann. Wenigstens eine Komponente des Verschlußelementes, also entweder der Ring oder die Kugel, besteht aus einem permanentmagnetischen Material. Die andere Komponente des Verschlußelementes besteht dann entweder aus einem ferromagnetischen Material oder aber ebenfalls aus einem permanentmagnetischen Material mit der entgegengesetzten Polung. In jedem Falle sorgt der magnetische Kraftschluß für das Streben der Kugel, in abdichtende Anlage gegen die Dichtfläche des Ringes zu kommen, wenn nicht eine Punktionskanüle durch die Portöffnung greift.

Zur Erhöhung der Dichtwirkung um ein Vielfaches ist vorteilhafterweise vorgesehen, den Ring in einen Silikonmantel einzubetten. So kommt ein Kontakt zwischen der Verschlußkugel und dem Ring mittelbar durch den Silikonmantel zum tragen, der sich im Bereich der Kontaktstellen leicht deformiert. Aufgrund der linienförmigen Anlage aber liegt in dem Bereich eine sehr hohe Hertzsche Pressung vor mit entsprechend hoher Dichtwirkung.

Nachteilig hieran könnte der relativ hohe Abrieb sein aufgrund der hohen Pressung, weswegen es gemäß einer anderen Ausführungsform vorgesehen ist, daß die Dichtfläche des Ringes gebildet ist aus einer am Silikonmantel angeformten umlaufenden Dichtlippe, die sich quasi an die Kugeloberfläche anschmiegt. Hier ist der Abrieb geringer aufgrund der wesentlich geringeren Werte für die Pressung. Andererseits ist die Dichtwirkung etwas herabgesetzt gegenüber dem vorerwähnten Ausführungsbeispiel. Sie reicht jedoch in jedem Falle noch mit Reserven aus.

Alternativ kann vorgesehen sein, daß die Dichtfläche an der innenliegenden Kante der Portöffnung im Silikonmantel als konvex abgerundete umlaufende Kante ausgebildet ist. Wiederum alternativ kann vorgesehen sein, daß die innenliegende Kante als konkav ausgehöhlte umlaufende Kante ausgebildet ist.

Bevorzugt besteht die permanentmagnetische Komponente des Verschlußelementes aus Seltenerden-Metall. Da dieses Metall per se nicht körperverträglich ist, muß der Ring aus einem solchen Material zwingend mit körperverträglichem Silikon bedeckt sein, darin eingebettet sein.

Eine Kugel aus einem Seltenerden-Metall ist zur Herstellung der Körperverträglichkeit bevorzugt mit einer Goldschicht versehen.

Der erfindungsgemäße Single-Needle-Port läßt sich auf einfache und zuverlässige Weise durch Einführen einer Punktionskanüle durch die Portöffnung hindurch öffnen. Beim Herausziehen der Punktionskanüle aus der Portöffnung sorgen die Magnetkräfte zwischen der Kugel und dem Ring für ein sofortiges sicheres Verschließen der Portöffnung.

Die Erfindung wird nachfolgend anhand eines Ausführungsbeispieles gemäß der Zeichnungsfiguren näher erläutert. Hierbei zeigt:
- Fig. 1: eine Schnittansicht durch den Single-Needle-Port, und
- Fig. 2: ebenfalls einen Schnitt durch den Single-Needle-Port, um 90° gegenüber der Fig. 1 gedreht.

Nachfolgend bezeichnen gleiche Teile dieselben Bezugszeichen.

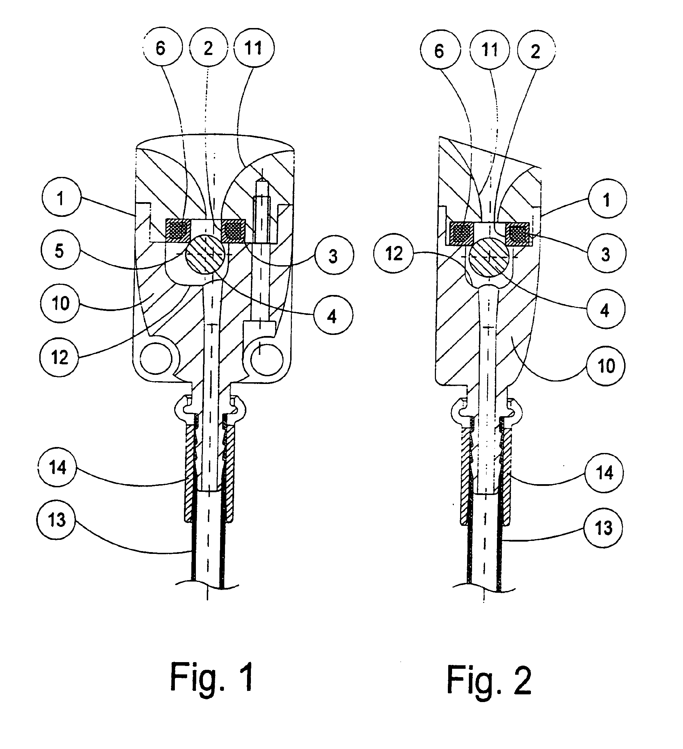

Fig. 1 zeigt den Schnitt durch den Single-Needle-Port 1. Diese besteht aus dem Grundkörper 10, der von außen durch den Einführtrichter 11 für eine Punktionskanüle (nicht dargestellt) zugänglich ist. Im Inneren ist der Ring 3 angeordnet, der vorliegend in einem körperverträglichen Silikon 6 eingebettet ist. In dichtender Anlage an dem Ring 3 sitzt als weiteres Verschlußelement die Verschlußkugel 4. Strichliniert ist deren Lage dargestellt, in welcher sie sich befindet, wenn eine Punktionskanüle durch die Portöffnung 2 greift. Dann nämlich wird die Verschlußkugel 4 durch die Portkanüle in eine im Inneren des Grundkörpers 2 ausgebildete Tasche 12 gedrängt. Wird die Punktionskanüle aus der Portöffnung 2 wieder herausgezogen, sorgen die magnetischen Kraftlinien zwischen der Verschlußkugel 4 und dem Ring 3 dafür, daß die Verschlußkugel 4 wieder in die dichtende Anlage an der unteren Kante der Portöffnung 2 gelangt.

Vorliegend sind die Dichtflächen des Silikons als rechtwinkelige Kanten dargestellt. Dies ergibt einen kreisförmigen Linienverlauf des Kontaktbereiches auf der Kugel 4 mit einer sehr hohen Hertzschen Pressung. Hieraus resultiert die enorme Dichtwirkung des Verschlußelementes aus dem Ring 3 und der Verschlußkugel 4.

Mit 13 ist symbolisch dargestellt das Blutgefäß, mit welchem der Single-Needle-Port 1 verbunden ist, vorliegend durch einen entsprechenden Adapter 14.

## Patentansprüche

1. Single-Needle-Port (1), der subkutan implantierbar ist und zur Durchführung einer Hämodialyse dient, bei dem die Portöffnung (2) mittels eines Verschlußelementes nach Beendigung der Hämodialyse abdichtbar ist und durch Einführung einer Punktionskanüle zur erneuten Behandlung zu öffnen ist, bei dem das Verschlußelement gebildet ist aus
- einem die Portöffnung (2) einfassenden Ring (3), und
- einer Verschlußkugel (4) mit einem größeren Durchmesser als die Portöffnung (2), die sich zu deren Abdichtung vom Portinneren her gegen eine Dichtfläche (5) des Ringes (3) anlegen kann und aus dieser Lage zur Freigabe des Lumens des Ports von einer in die Portöffnung (2) zu führenden Punktionskanüle verschoben werden kann,
**dadurch gekennzeichnet,**
**daß** wenigstens eine Komponente des Verschlußelementes aus permanentmagnetischem Material und die andere Komponente aus einem ferromagnetischen Material bzw. aus einem permanentmagnetischen Material entgegengesetzter Polung besteht, und daß der Ring (3) in einem Silikonmantel (6) eingebettet ist.

2. Single-Needle-Port nach Anspruch 1 bei dem die Komponente aus permanentmagnetischem Material aus Seltenerden-Metall besteht.

3. Single-Needle-Port nach Anspruch 1 oder 2, bei dem die Kugel (4) mit einer Goldschicht belegt ist.

4. Single-Needle-Port nach einem der Ansprüche 1 bis 3, bei dem die Dichtfläche (5) des Ringes (6) gebildet ist aus einer am Silikonmantel (6) angeformten umlaufenden Dichtlippe.

5. Single-Needle-Port nach einem der Ansprüche 1 bis 3, bei dem die Dichtfläche (5) an der innenliegenden Kante der Portöffnung (2) im Silikonmantel (6) als konvex abgerundete umlaufende Kante ausgebildet ist.

6. Single-Needle-Port nach einem der Ansprüche 1 bis 3, bei dem die Dichtfläche (5) an der innenliegenden Kante der Portöffnung (2) im Silikonmantel (6) als konkav ausgehöhlte umlaufende Kante ausgebildet ist.

7. Single-Needle-Port nach einem der Ansprüche 1 bis 3, bei dem die Dichtfläche (5) an der innenliegenden Kante der Portöffnung (2) als rechtwinklige Kante ausgebildet ist.

## Claims

1. Single-needle port (1) which can be implanted subcutaneously and serves for carrying out haemodialysis, in which the port opening (2) can be sealed by means of a closure element after completing haemodialysis and can be opened by introducing a puncture cannula for renewed treatment, in which the closure element is formed from
- a ring (3) bordering the port opening (2), and
- a closure ball (4) having a greater diameter than the port opening (2), which for sealing thereof from the port interior, may rest against a sealing surface (5) of the ring (3) and may be displaced from this position to release the lumen of the port from a puncture cannula to be guided into the port opening (2),
**characterised in that** at least one component of the closure element consists of permanent-magnetic material and the other component consists of a ferromagnetic material or of a permanent-magnetic material of opposite polarity, and **in that** the ring (3) is embedded in a silicone shell (6).

2. Single-needle port according to claim 1, in which the component consists of permanent-magnetic material made from rare earth metal.

3. Single-needle port according to claim 1 or 2, in which the ball (4) is covered by a gold layer.

4. Single-needle port according to one of claims 1 to 3, in which the sealing surface (5) of the ring (6) is formed from an annular sealing lip moulded onto the silicone shell (6).

5. Single-needle port according to one of claims 1 to 3, in which the sealing surface (5) is designed as a convexly rounded-off annular edge on the inner-lying edge of the port opening (2) in the silicone shell (6).

6. Single-needle port according to one of claims 1 to 3, in which the sealing surface (5) is designed as a concavely hollowed-out annular edge on the inner-lying edge of the port opening (2) in the silicone shell (6).

7. Single-needle port according to one of claims 1 to 3, in which the sealing surface (5) is designed as a right-angled edge on the inner-lying edge of the port opening (2).

## Revendications

1. Voie d'accès à aiguille unique (1), qui peut être implantée par voie sous-cutanée et qui sert à réaliser une hémodialyse, dans laquelle l'ouverture de la voie d'accès (2) peut être étanchéifier au moyen d'un élément obturateur une fois l'hémodialyse terminée et peut être ouverte par introduction d'une canule de ponction en vue d'un nouveau traitement, dans laquelle l'élément obturateur est constitué
- d'une bague (3) entourant l'ouverture de la voie d'accès (2) et
- d'une bille d'obturation (4) ayant un diamètre supérieur à l'ouverture de la voie d'accès (2), qui pour l'étanchement de celle-ci peut s'appuyer depuis l'intérieur de la voie d'accès contre une surface d'étanchéité (5) de la bague (3) et qui, à partir de cette position, peut être déplacée par une canule de ponction à guider dans l'ouverture de la voie d'accès (2) afin de libérer la lumière de la voie d'accès,
**caractérisée en ce qu'**au moins un composant de l'élément obturateur se compose d'un matériau magnétique permanent et l'autre composant d'un matériau ferromagnétique et/ou d'un matériau magnétique permanent de polarité opposée, et **en ce que** la bague (3) est encastrée dans une enveloppe de silicone (6).

2. Voie d'accès à aiguille unique selon la revendication 1 dans laquelle le composant constitué de matériau magnétique permanent se compose de métal des terres rares.

3. Voie d'accès à aiguille unique selon la revendication 1 ou 2, dans laquelle la bille (4) est recouverte d'une couche d'or.

4. Voie d'accès à aiguille unique selon l'une quelconque des revendications 1 à 3, dans laquelle la surface d'étanchéité (5) de la bague (3) est formée à partir d'une lèvre d'étanchéité circulaire rapportée par formage sur l'enveloppe de silicone (6).

5. Voie d'accès à aiguille unique selon l'une quelconque des revendications 1 à 3, dans laquelle la surface d'étanchéité (5) est formée comme arête circulaire arrondie de façon convexe au niveau de l'arête intérieure de l'ouverture de la voie d'accès (2) dans l'enveloppe de silicone (6).

6. Voie d'accès à aiguille unique selon l'une quelconque des revendications 1 à 3, dans laquelle la surface d'étanchéité (5) est formée comme arête circulaire creusée de façon concave au niveau de l'arête intérieure de l'ouverture de la voie d'accès (2) dans l'enveloppe de silicone (6).

7. Voie d'accès à aiguille unique selon l'une quelconque des revendications 1 à 3, dans laquelle la surface d'étanchéité (5) est formée comme arête orthogonale au niveau de l'arête intérieure de l'ouverture de la voie d'accès (2).
